# EUROPEAN PATENT APPLICATION

(11) **EP 3 815 659 A1**
(43) Date of publication of application: **05.05.2021**
(21) Application number: 20204501.9
(22) Date of filing: 28.10.2020
(51) Int. Cl.: A61F 13/53, B32B 27/08, B32B 27/16, B32B 27/30, C08F 220/20, C09D 133/06, C09D 133/14

(54) **IPN HYDROGEL FOR PREPARATION AND APPLICATION**

(30) Priority: 30.10.2019 TW 108139285
(71) Applicant: Easting Biotechnology Company Limited, New Taipei City 22102 (TW)
(72) Inventor: Hsieh, Meng yow, 22102 New Taipei City (TW); Chen, Shih-Wei, 22102 New Taipei City (TW); Yang, Shiu-Feng, 22102 New Taipei City (TW); Lee, Huan-Cheng, 22102 New Taipei City (TW); Liu, Yen-Chu, 22102 New Taipei City (TW)
(74) Representative: Lang, Christian

(57) **Abstract**

The invention discloses an interpenetrating biopolymers network (IPN) hydrogel loaded with herbal extracts, preparation and application thereof. The IPN hydrogel can increase the preservation time of herbal extracts and achieve a long-term release mechanism of herbal extracts. The interpenetrating biopolymers network (IPN) hydrogel makes itself have microporous and macroporous network structure and reinforced gel structure. The gel itself has good hydrophilicity and high biocompatibility.

In addition, the present invention will be subsequently applied to the development of hydrogel patches and preparation methods, which are composed of bidirectional elastic non-woven fabrics, hydrogels containing extracts and a cover film layer, and solve the common the problem of allergies caused by patches.

## Description

### FIELD OF THE INVENTION

The present invention is an interpenetrating biopolymer network hydrogel and its preparation method. It belongs the field of biological medical material.

### BACKGROUND OF THE INVENTION

Biopolymers hydrogel is three-dimensional interpenetrating crosslink network which is composed of nature substance such are protein and polysaccharide that can keep a large amount of water when it is swelling in water. Because biopolymers hydrogel has rich water environment, good biocompatibility and viscoelasticity similar to the structure of biological tissue and that can provide good transmission channels for diffusion of nutrition and biological active ingredient. Its excellent water absorption, water retention and biomimetic properties make biopolymers hydrogel widely be used in the field of biomedical material and tissue engineering. It even can be tissue filling material, drug-controlled release carriers, artificial skin, artificial cartilage, tissue engineering scaffold materials. However, the mechanical strength of hydrogels, especially biopolymers hydrogel, is generally low, which severely restricts its practical application in artificial skin, artificial cartilage and tissue engineering scaffold materials.

The traditional covering structure is composed of natural plant fiber or animal hair materials, such as gauze, cotton pad, wool, various oil gauze, etc., these kind of covering structure are only temporary covering materials, all of them need to be replaced within a period of time. However, these wound patch structures are prone to stick to the wound when they are replaced. Therefore, when the patch structure is torn off, it is possible to tear open new epithelial cells or wounds that have gradually healed. That causes pain and it is difficult for users to bear, and is not conducive to the natural healing of the wound.

Interpenetrating biopolymers network hydrogel is a unique type of network interpenetrating polymer formed by physical or chemical cross-link and entanglement of two or more polymers. Through the form of network interpenetration, two polymers with different functions can form a stable combination, thereby achieving complementary performance between components; its structural characteristics such as interpenetration and bidirectional continuity of the interface make them better in performance or function. Produce special synergies. Compared with block copolymers, the phase morphology of these systems is relatively stable to environmental changes because it is fixed by crosslinking. Therefore, preparing an interpenetrating network is one of the most effective ways to improve the strength of hydrogel.

In order to overcome the low mechanical strength of biopolymers hydrogel, using the structure of interpenetrating network to solve mechanical problems and it also extends the time-releasing of medications. The material of hydrogel in the present invention is 2-Hydroxyethyl methacrylate (HEMA) which is used for contact lenses as main monomer. By adjusting the composition of monomer, the structure of hydrogel can be improved and assess the drug system.

### DETAILED DESCRIPTION OF THE INVENTION

The purpose of the present invention which provides a high mechanism strength biopolymers hydrogel and preparation method. The hydrogel has excellent mechanical properties and biocompatibility. In addition, the present invention applies to development of hydrogel patches and preparation method, which was composed of bidirectional elastic non-woven fabric, hydrogel containing extracts and cover film layer, to solve common allergic phenomenon problem which patches caused.

IPN (interpenetrating polymer network) is a state or structure of cross-linked polymer, which was synthesized from at least one monomer or crosslinked another monomer with cross-linking agent. There is not covalent bond between each other, only when the chemical bond was broken, the monomers of the polymers could be separated.

The present invention was ethylenically unsaturated monomers as materials of the IPN hydrogel. According to different type of monomer, the structure of the IPN hydrogel, time-releasing of medications and water absorption property were improved.

An interpenetrating biopolymers network hydrogel comprising:a first polymer layer; a second polymer layer; wherein the first polymer layer and the second polymer layer are respectively formed by polymerizing at least one alkaline treated ethylenically unsaturated monomer, crosslink through a cross-linking agent and a photoinitiator; and the pH value of the hydrogel is 6.5 to 8.

The ethylenically unsaturated monomer used in the IPN hydrogel of the present invention is selected from the group consisting of hydroxyethyl acrylate (HEA), hydroxyethyl methacrylate (HEMA), 2-hydroxypropyl acrylate (2-HPA), 2-hydroxypropyl methacrylate (2-HPMA), 3-hydroxypropyl acrylate (3-HPA), 3-hydroxypropyl methacrylate (3-HPMA), acrylic-2,3-dihydroxypropyl ester, 2,3-dihydroxypropyl methacrylate, 1,3-dipropenylglycerol, 1,3-dimethylpropenylglycerol, trimethylolpropane monoacrylate, trimethylolpropane monomethacrylate, trimethylolpropane diacrylate, trimethylolpropane dimethacrylate, 2-propenamido-2-methyl-1-propanesulfonic acid (AMPS), or the combination thereof.

In the preferred embodiment, the IPN hydrogel used hydroxyethyl methacrylate (HEMA) and 2-propenamido-2-methyl-1-propanesulfonic acid (AMPS) as the main monomers, and added photoinitiator.

The photoinitiator used in the present invention is α-ketoglutarate (α-KGA) or 2,2-diethoxy acetophenone (DEAP), or 2-hydroxy-2-methyl-1-Phenyl-1-acetone (HMPP).

The cross-linking agent which the present invention used is N,N'-methylene-bisacrylamide (NMBA), ethylene glycol di(meth)acrylate, 1,4-diacrylic acid piper (PDA), glutaraldehyde, epichlorohydrin, or a combination thereof.

The present invention is IPN hydrogel, wherein the cross-linking agent is photo cross-linking agent.

The pH value in the present invention is 6.5 to 8.

In the preferred embodiment, the pH value in the present invention is 7.4 to 7.8.

AMPS should be neutralized with NaOH before using it. The prepared pre-polymerization solution should be measured pH value. The pH value should be adjusted with weak acid or weak base before polymerization. 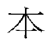 The present invention can be applied to biomedical applications. First the hydrogel itself does not stick to the wound, and has high absorption to lock water and moisturize, maintain a moist and balanced environment, and accelerate wound healing. The hydrogel is translucent that is available to observe the change if the permeate oozes. The hydrogel itself is more comfortable to wear and breathable than the commercially available pressure sensitive adhesive.

### BRIEF DESCRIPTION OF THE DRAWINGS

Fig.1 shows a schematic diagram of the cross-linked method of present invention.
Fig.2 shows a preparation principle of the present invention.
Fig.3 shows a schematic diagram of structure of the present invention.
Fig.4 shows a release curve of the hydrophilic substance of the present invention.
Fig.5 shows a release curve of lipophilic substances of the present invention.

[ 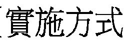 ]

### Example 1

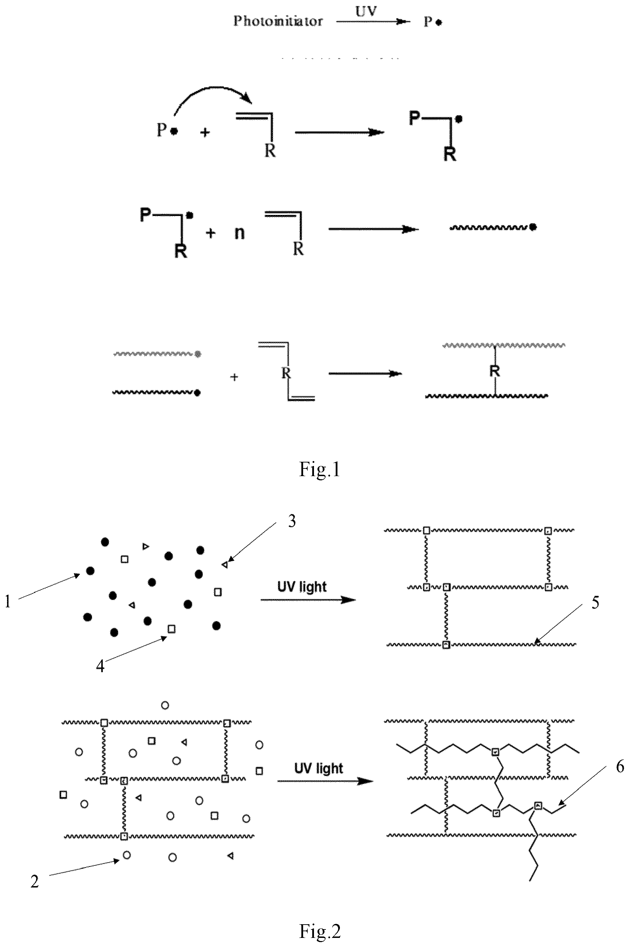

Fig.1 shows the photoinitiator would generate free radicals through UV irradiation and the free radicals would attack monomer or the vinyl group on the cross-linking agent, which made them to generate new free radicals. When new free radicals continuously contacted with monomer, it would initiate a continuous chain extension reaction to form a polymer. Among them, a cross-linked monomer had more than two vinyl groups. When more than two vinyl groups formed free radicals separately and connected with two different polymer chains to form cross-link reaction.

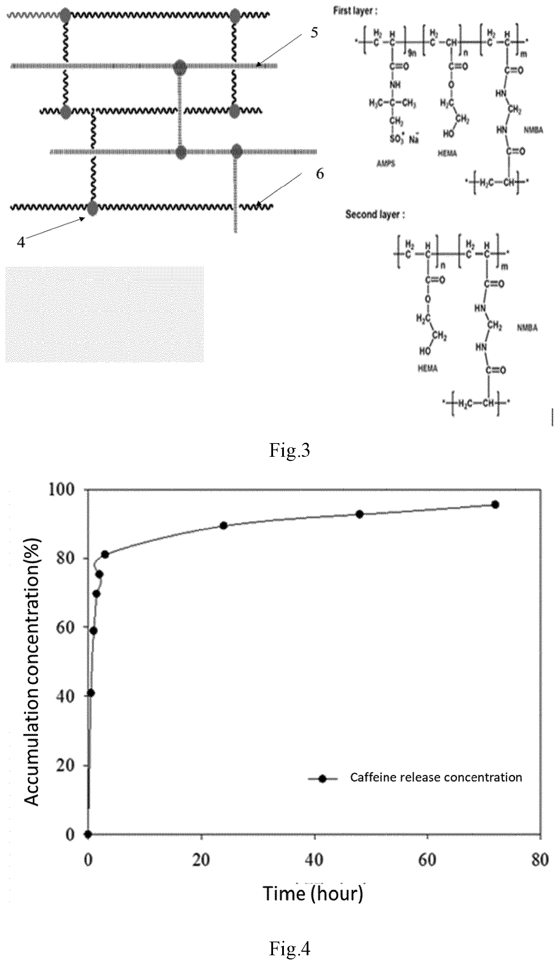

Figs.2 and 3 illustrate that present invention is a preparation method of interpenetrating biopolymers network, comprising dissolving hydroxyethyl methacrylate (HEMA) (1) and 2-propenamido-2-methyl-1-propanesulfonic acid (AMPS) (2) in water according to a specific ratio, adding the photo initiator α-ketoglutaric acid (α-KGA) (3) and the cross-linking agent (4) N,N'-methylenebisacrylamide (NMBA). After mixing them uniformly, injecting with a syringe to prepare in advance in a good glass mold; placing the mold under an ultraviolet light source for photopolymerization. After exposure to UV light for a period of time, the mold is taken out and removed it to obtain the first layer hydrogel. Prepare another mixed solution which comprises HEMA, AMPS, α-KGA and NMBA with a specific concentration ratio, soaking the first layer gel (5) made it swell, and after the first layer gel was completely swelled, place it under the ultraviolet light source to make second layer gel (6) photopolymerize. After the reaction was completed, the target interpenetrating network gel could be obtained.

Table 1 is the implementation process of the present invention that shows the IPN gel at different concentrations, the weight of different monomers and the ratio of cross-linking agent. Production process: dissolving the neutralized AMPS and HEMA in a solvent according to the composition ratio in Table 1, adding the cross-linking agent NMBA and the photo initiator α-KGA in sequence. After mixing them uniformly, adjusting the concentration to the target value. Using syringe injected into the glass mold and placed under an ultraviolet light source for photopolymerization. After the reaction was completed, the mold was removed and obtain the first layer gel. Then, the neutralized AMPS, HEMA, NMBA, and α-KGA were configured into the second layer gel solution according to the ratio in Table 1. The first layer gel was immersed in the second layer gel solution for swelling, and after it was completely swelled, it was taken out and placed under an ultraviolet light source for the second photopolymerization reaction. After the reaction was completed, the cross-linked hydrogel of the interpenetrating network was obtained.

**Table 1**

| First layer of gel | | | | | |
|---|---|---|---|---|---|
| number | concentration (M) | HEMA (mol%) | AMPS (mol%) | NMBA (mol%) | Photoinitiator (mol%) |
| H10A0 | 3 | 100 | 0 | 3 | 0.5 |
| H95A5 | | 95 | 5 | 3 | 0.5 |
| H50A50 | | 50 | 50 | 3 | 0.5 |
| H5A95 | | 5 | 95 | 3 | 0.5 |
| H0A100 | | 0 | 100 | 3 | 0.5 |

| Second layer of gel | | | | | |
|---|---|---|---|---|---|
| number | concentration (M) | HEMA (mol%) | AMPS (mol%) | NMBA (mol%) | Photoinitiator (mol%) |
| HEMA | 0.8 | 90 | 10 | 0.5 | 0.5 |

The drug release test process of the present invention

### Hydrophilic drug release

After the first layer gel was completed, the first layer gel was subsequently immersed in the second layer gel solution for swelling. At this time, the second layer gel solution was mixed with drugs. After it completely swelled, it is taken out and placed under the ultraviolet light source to carry out the second photopolymerization reaction.

The drug-containing hydrogel was placed in a sustained-release solution for drug release testing and taking the samples from sustained-release solution within a fixed time.

Taking the samples with fixed time and test releasing concentration. The sampling time is 30 minutes, 60 minutes, 90 minutes, 180 minutes, 8 hours, 24 hours, 48 hours, and 72 hours.

The subsequent drug concentration was analyzed by high performance liquid chromatography (HPLC). The water-based drug used caffeine for drug release, and the absorption wavelength of caffeine was 272 nm for measurement.

Fig.4 shows the caffeine is water-soluble compound. It can be known from the release curve that the release amount of caffeine in water was 40% and above.

### Lipophilic drug release

After the first layer gel was completed, the first layer gel was subsequently immersed in the second layer gel solution for swelling. At this time, the second layer gel solution is mixed with drugs. After it completely swelled, it was taken out and placed under the ultraviolet light source to carry out the second photopolymerization reaction.

The drug-containing hydrogel was placed in a sustained-release solution for drug release testing and taking the samples from sustained-release solution within a fixed time.

Taking the samples with fixed time and testing its releasing concentration. The sampling time is 15 minutes, 30 minutes, 45 minutes, 60 minutes, 90 minutes, 120 minutes, and 180 minutes.

The subsequent drug concentration was analyzed by high performance liquid chromatography and the lipophilic drug was measured with the absorption wavelength of the lipophilic dye at 210 nm.

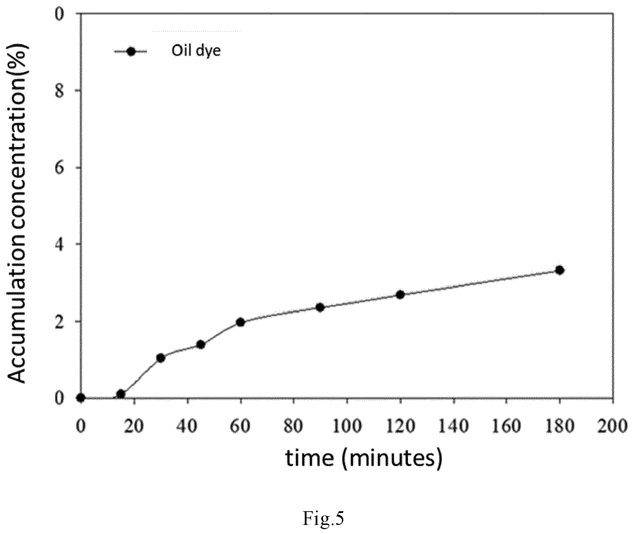

Fig.5 is release curve shows that the release rate of the lipophilic substance which stored the hydrogel carrier in water was 3% and above.

The above-mentioned embodiments merely illustrate the effects of the present invention and the technical features of the present invention does not use to limit the protection scope of the present invention. Any change or arrangement can be easily made by a person skilled in the art without departing from the technical principle and spirit of the present invention and these are the scope of the present invention. Therefore, the protection scope of the present invention is as listed in the attached patent scope.

## Claims

1. An interpenetrating biopolymers network hydrogel comprising:
a first polymer layer;
a second polymer layer;
wherein the first polymer layer and the second polymer layer are respectively formed by polymerizing at least one alkaline treated ethylenically unsaturated monomer, crosslink through a cross-linking agent and a photoinitiator; and the pH value of the hydrogel is 6.5 to 8.

2. The interpenetrating biopolymers network hydrogel according to claim 1, wherein the ethylenically unsaturated monomer is selected from the group consisting of hydroxyethyl acrylate (HEA), hydroxyethyl methacrylate (HEMA), 2-hydroxypropyl acrylate (2-HPA), 2-hydroxypropyl methacrylate (2-HPMA), 3-hydroxypropyl acrylate (3-HPA), 3-hydroxypropyl methacrylate (3-HPMA), acrylic-2,3-dihydroxypropyl ester, 2,3-dihydroxypropyl methacrylate, 1,3-dipropenylglycerol, 1,3-dimethylpropenylglycerol, trimethylolpropane monoacrylate, trimethylolpropane monomethacrylate, trimethylolpropane diacrylate, trimethylolpropane dimethacrylate, 2-propenamido-2-methyl-1-propanesulfonic acid (AMPS), or the combination thereof.

3. The interpenetrating biopolymers network hydrogel according to claim 2, wherein the ethylenically unsaturated monomer are hydroxyethyl methacrylate (HEMA), and 2-propenamido-2-methyl-1-propanesulfonic acid (AMPS).

4. The interpenetrating biopolymers network hydrogel according to claim 1, wherein the photoinitiator is α-ketoglutarate (α-KGA), 2,2-diethoxy acetophenone (DEAP), or 2-hydroxy-2-methyl-1-Phenyl-1-acetone (HMPP).

5. The interpenetrating biopolymers network hydrogel according to claim 1, wherein the cross-linking agent is N,N'-methylene-bisacrylamide (NMBA), ethylene glycol di(meth)acrylate, 1,4-diacrylic acid piper (PDA)), glutaraldehyde, epichlorohydrin, or a combination thereof.

6. The interpenetrating biopolymers network hydrogel according to claim 1, wherein the cross-linking agent is photo cross-linking agent.

7. The interpenetrating biopolymers network hydrogel according to claim 5, wherein the cross-linking agent is N,N'-methylene-bisacrylamide (NMBA).

8. The interpenetrating biopolymers network hydrogel according to claim 1, wherein the pH value is 7.4 to7.8.

9. The interpenetrating biopolymers network hydrogel according to claim 1, wherein the hydrophilic substance release rate is 40% and above.

10. The interpenetrating biopolymers network hydrogel according to claim 1, wherein the lipophilic substance release rate is 3% and above.
